(19) Europäisches Patentamt
European Patent Office
Office européen des brevets

(11) Publication number: **0 059 057**
**A1**

(12) **EUROPEAN PATENT APPLICATION**

(21) Application number: **82300758.8**

(22) Date of filing: **16.02.82**

(51) Int. Cl.³: **A 61 K 31/70**, A 61 K 33/00
//
(A61K33/00, 31/70, 31/195, 31/135)

(30) Priority: **20.02.81 GB 8105340**
**28.02.81 GB 8106416**
**12.03.81 GB 8107785**

(43) Date of publication of application: **01.09.82**
**Bulletin 82/35**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Newsome, Peter Martin, 41 St Clair Drive, Worcester Park Surrey (GB)**
Inventor: **Burgess, Malcolm Neil, 17 Englefield Hills Farm Lane, Horsham Surrey (GB)**
Inventor: **Richards, David Hugh, 'Calgary' Bentbrook Park North Holmwood, Dorking Surrey (GB)**
Inventor: **Mullen, Noel Austin, 3 Rowland Road, Cranleigh Surrey (GB)**

(74) Representative: **Cresswell, Thomas Anthony et al, European Patent Attorney Beecham Pharmaceuticals Great Burgh Yew Tree Bottom Road, Epsom Surrey KT18 5XQ (GB)**

(54) **Treatment of diarrhoea.**

(57) $\alpha_2$-adrenergic agonists, especially compounds (I)

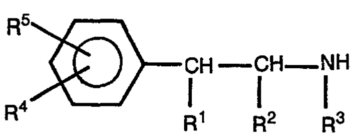

(I)

wherein
$R^1$ is hydrogen or hydroxy
$R^2$ is hydrogen or methyl
$R^3$ is hydrogen or methyl
$R^4$ is hydrogen, meta-hydroxy or orth-methoxy and
$R^5$ is hydrogen, para-hydroxy or meta-methoxy
and salts thereof
may be administered orally for treatment of diarrhoea and scours in association with oral rehydration formulas.

EP 0 059 057 A1

# Treatment of Diarrhoea

The present invention relates to the treatment of diarrhoea and to compositions for use in such treatment.

Diarrhoea (also referred to as scours in cattle, sheep and pigs) can be a severe disease particularly in young animals and can often result in death. Diarrhoea is also common amongst human travellers and those exposed to low standards of hygiene. The diarrhoea frequently involves colonisation of the small intestine with enteropathogenic strains of E. coli which produce heat stable and/or heat labile enterotoxins. Related enterotoxins are produced by other enteropathogens, for example, cholera, and also cause diarrhoea. These enterotoxins stimulate fluid secretion in the gut lumen and hence cause diarrhoea. The associated fluid loss may lead to loss of condition, reduced weight gain of livestock and often to death.

It has now been found that a class of sympathomimetic amines and salts thereof have properties useful in the treatment of diarrhoea. In particular the compounds reduce dehydration caused by hypersecretory diarrhoeas, such as E. coli scours, when administered orally.

Accordingly the present invention provides a method for treating diarrhoea in humans and other mammals which method comprises the oral administration

to a human or other mammal suffering from diarrhoea of an effective, non-toxic amount of a sympathomimetic phenylethylamine derivative having $\alpha_2$-adrenoceptor agonist activity.

According to a particular aspect the present invention provides a method for treating diarrhoea which method comprises the oral administration to a human or other mammal suffering from diarrhoea of a compound of formula (I):

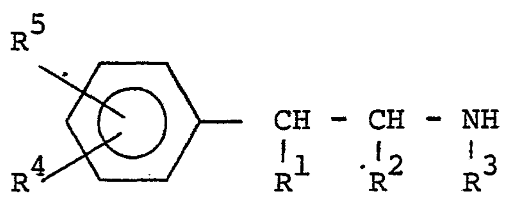

(I)

wherein $R^1$ is hydrogen or hydroxy
$R^2$ is hydrogen or methyl
$R^3$ is hydrogen or methyl
$R^4$ is hydrogen, meta-hydroxy or orthomethoxy
$R^5$ is hydrogen, para-hydroxy or meta-methoxy
or a salt thereof provided that the compound has some $\alpha_2$-adrenergic agonist activity.

As used herein oral administration includes administration <u>via</u> a stomach tube. An $\alpha_2$-adrenergic agonist is a compound which is capable of displacing some tritiated clonidine from specific receptors in brain preparations.

For example the compound is epinephrine, norepinephrine, nordefrin, metaraminol, phenylephrine,

hydroxyamphetamine, methoxamine, methamphetamine, ephidrine, phenylpropanolamine and mephentermine.

Preferably the compound is epinephrine, norepinephrine or metaraminol. Suitable salts include the mono- and di-salts of the following acids, i.e. hydrochloric, hydrobromic, hydroiodic, tartaric, citric and ascorbic acid.

It has been found particularly advantageous to combine the above method of treatment with oral rehydration therapy.

Accordingly the present invention provides, in a particular aspect, a method for treating diarrhoea which method comprises the oral administration to a human or other mammal suffering from diarrhoea of an effective non-toxic amount of a phenylethylamine derivative especially a compound of formula (I) as hereinbefore defined and an oral rehydration composition comprising a pharmacologically acceptable aqueous solution containing at least 0.5% w/v of an actively absorbed monosaccharide, at least 25 m<u>M</u> sodium ions and having an osmolarity less than 500 m Osmolar.

Preferably the oral rehydration composition further comprises actively-absorbed amino acids and electrolytes.

Active absorption (or active transport) is well known to the skilled man, as are the monosaccharides and amino acids which are actively absorbed. In this regard the reader is referred to standard text books such as 'Medicinal Physiology' by Guyton (published by W.B. Saunders and Company) 4th Edition pages 769 to 771. Of course whether or not a particular monosaccharide or amino acid is actively absorbed may also readily be determined by experiment as for example described in Wilson T.H. 1962 Intestinal Absorption (Saunders, Philadelphia).

To be actively absorbed, monsaccharides must have (a) at least six carbon atoms in their chain (b) a D-pyranose ring structure and (c) an intact hydroxyl group at carbon 2. Thus suitable examples of monosaccharides for use in this invention include the naturally occurring D-pyranoses such as glucose and galactose. Other examples of suitable monosaccharides include naturally occurring D-pyranoses that have been chemically modified whilst retaining the necessary structural features (a), (b) and (c). Examples of such modified monosaccharides include $C_{2-7}$ acylated and $C_{1-4}$ alkylated derivatives, such as acetyl, methyl, ethyl and n- and iso-propyl derivatives. Specific examples include α-methyl glucoside, 3-O-methyl glucose and 6-deoxygalactose.

Preferably the monosaccharide will be glucose or galactose. The monosaccharide of choice for use in this invention is glucose (e.g. dextrose).

Suitable examples of actively absorbed naturally occurring amino acids include neutral amino acids such as glycine and alanine and basic amino acids such as arginine. Preferably the amino acid is glycine.

Suitable electolytes for such inclusion include salts containing ions such as sodium, potassium, calcium, magnesium, chloride, phosphate, gluconate, sulphate, bicarbonate, carbonate and the like. Other favoured electrolytes for inclusion in the compositions include potassium dihydrogen phosphate, dipotassium hydrogen phosphate, tripotassium phosphate, potassium chloride and the like, with potassium dihydrogen phosphate being particularly suitable.

One particularly preferred electolyte for inclusion in the composition of the invention is sodium chloride.

Suitably the sympathomimetic phenylethylamine derivative, is administered in solution in the oral rehydration composition. However it may also be administered separately in an orally administrable form.

Suitably the compound of formula (I) is administered at from 100 μg/kg/day to 40 mg/kg/day depending on the activity of the compound and the recipient species, for instance 1 to 5 mg/kg/day most conveniently in two equal doses.

For use in the above treatments, the compound of formula (I) may be presented as a pharmaceutically acceptable composition.

Accordingly the present invention provides an orally administrable pharmaceutical composition comprising a sympathomimetic phenylethylamine derivative having $\alpha_2$ adrenoceptor agonist activity, which is, preferably, a compound of formula (I), and a pharmaceutically acceptable carrier therefor.

As used herein the term "pharmaceutically acceptable" includes veterinarily acceptable.

In particular, the present invention provides an improved oral rehydration formulation comprising an actively absorbed monosaccharide, sodium ions and a sympathomimetic phenylthylamine derivative preferably compound of formula (I) as hereinbefore defined.

The improved oral rehydration formulation may be presented as a dry powder for dissolution in water or a concentrate for dilution in water. Alternatively the formulation may be an orally administrable solution in water, in which case the solution contains at least

0.5% w/v of the monosaccharide and at least 25 mM sodium ions and has an osmolarity of less than 500 m Osmolar. Preferably the formulation also comprises an actively absorbed amino acid and electrolyles

Most preferably the formulation comprises a compound of formula (I) and an oral rehydration formulation as described in U.K. Patent No. 1 581 826 and U.K. Patent Application No. 2 012 163 A, U.S. Patent No. 3 898 328 or Nalin, D.R. & Cash, R.A., Bull World Health Org, 43, 361, (1970).

Advantageously the improved formulation includes a preservative or antioxidant such as ascorbate anions or sodium metabisulphite. When presented in solution the formulation would preferably comprise about 6 mM ascorbate or up to 0.1% w/v sodium metabisulphite.

Advantageously the formulation, when presented as a dry powder, is packaged to exclude air and moisture.

The present invention will now be illustrated with reference to the following Examples, which are not intended to limit the scope of the invention in any way.

Example 1

15 g of the following composition may be prepared by mixing together the ingredients in dry powder form:

| | | |
|---|---|---|
| Adrenaline | : | 0.5% by weight |
| Dextrose (anhydrous) | : | 71.5% by weight |
| Glycine | : | 12% by weight |
| Sodium Chloride | : | 7% by weight |
| Potassium Dihydrogen Phosphate | : | 7% by weight |
| Citric Acid | : | 2% by weight |

The resultant unit-dose composition is dissolved in 500 ml of water. Adrenaline bitartrate may be used instead of adrenalin.

<u>Example 2</u>

8 g of the following composition may be prepared by mixing together the ingredients in dry powder form:

| | | |
|---|---|---|
| Adrenaline | : | 0.3% by weight |
| Dextrose (anhydrous) | : | 66.7% by weight |
| Glycine | : | 10.3% by weight |
| Sodium Chloride | : | 14.3% by weight |
| Potassium dihydrogen phosphate | : | 6.8% by weight |
| Citric Acid | : | 0.8% by weight |
| Tripotassium citrate | : | 0.2% by weight |

The resultant unit-dose composition is then dissolved in 250 ml of water. Adrenalin bitartrate may be used instead of adrenaline.

Example 3

The procedures of Examples 1 and 2 may be repeated, but prior to dissolution in water the ingredients are filled into a twin sachet with the dextrose in one part of the sachet and all the other ingredients in the other part of the sachet.

Example 4

Each of Examples 1 to 3 may be repeated with otherwise identical compositions containing an orange flavour.

Example 5

Each of Examples 1 to 3 may be repeated with otherwise identical compositions containing a lemon flavour.

Example 6

Twin sachets may be filled with the following ingredients:

Sachet A

| Ingredient | Weight g. | Particle Size μ |
|---|---|---|
| Glycine U.S.P. | 0.7725 | 500 |
| Citric acid (anhydrous) Ph. Eur. | 0.0600 | 370 |
| Potassium dihydrogen phosphate N.F. | 0.5100 | 350 |
| Potassium citrate Ph. Eur. | 0.0150 | milled to $<$50 |
| Sodium chloride Ph. Eur. | 1.0725 | 365 |
| Adrenaline | 0.0250 | |
| | 2,4550 g. | |

Sachet B

| Ingredient | Weight g. |
| --- | --- |
| Dextrose monohydrate B.P. | 4.2315 |
| Aerosil 200 (Colloidal Silicon Dioxide N.F.) | 0.0059 |
| Orange dry flavour, dextrose based, containing 75% dextrose as monohydrate | 0.9960 |
| Lemon dry flavour dextrose based, containing 90% dextrose as monohydrate | 0.6628 |
| | 5.8962 g |

(Total dextrose monohydrate weight is 5.5750 g.)

The twin sachets thus formed are then opened and their contents dissolved in 250 ml. of water.

Example 7

1 kg of the following composition may be prepared by mixing together the ingredients in dry powder form:

| | | |
|---|---|---|
| Adrenaline | : | 0.1% |
| Glycine | : | 10.3 |
| Dextrose (anhydrous) | : | 67.5 |
| Sodium Chloride | : | 14.3 |
| Potassium Dihydrogen Phosphate | : | 6.8 |
| Citric Acid | : | 0.8 |
| Tri-potassium Citrate | : | 0.2 |

60 g of the composition is then dissolved in 2 litres of water. Adrenaline bitartrate may be used instead of adrenaline.

Example 8

The following composition may be prepared by a method analogous to that of Example 7:

| | | |
|---|---|---|
| Glycine | : | 10% |
| Adrenaline | : | 0.1 |
| Dextrose (anhydrous) | : | 71.9 |
| Sodium Chloride | : | 10 |
| Citric Acid | : | 5 |
| Tri-potassium Citrate | : | 3 |

60 g. of the composition is then dissolved in 2 litres of water. Adrenaline bitartrate may be used instead of adrenaline.

Example 9

For storage, the composition according to Example 7 is prepared in the same manner, but the dextrose (676 g.) was filled into one container and the remaining ingredients (324 g.) were filled into a second container.

Example 10

The following formulation may be prepared by a method analogous to that of Example 7.

| | w/w % |
|---|---|
| Sodium Chloride | 11.6 |
| Calcium Gluconate | 2.2 |
| Magnesium Sulfate | 0.6 |
| Monopotassium Phosphate | 8.7 |
| Glycine | 21.2 |
| Adrenaline | 0.1 |
| Dextrose, anhydrous | 55.6 |

Example 11

1 k.g. of the following composition may be prepared by mixing together the ingredients in dry powder form:

| | | |
|---|---|---|
| Adrenaline | : | 0.1% |
| Glycine | : | 10.3% |
| Dextrose (anhydrous) | : | 67.5 |
| Sodium Chloride | : | 14.3 |
| Potassium Dihydrogen Phosphate | : | 6.8 |
| Tri-potassium Citrate | : | 1.0 |

60 g. of the composition is then dissolved in 2 litres of water.

Example 12

The following composition may be prepared by a method analogous to that of Example 11.

| | | |
|---|---|---|
| Adrenaline | : | 0.1% |
| Glycine | : | 10 |
| Dextrose (anhydrous) | : | 71.9 |
| Sodium Chloride | : | 10 |
| Tri-potassium Citrate | : | 8 |

60 g. of this composition is then dissolved in 2 litres of water.

Example 13

For storage, the composition according to Example 11 is prepared in the same manner, but the dextrose (676 g.) is filled into one container and the remaining ingredients (324 g.) are filled into a second container.

Example 14

To 60 g. of a composition prepared according to Example 7 is added 400 mg. of amoxycillin.

Example 15

1 k.g. of each of the following compositions may be prepared by mixing together the ingredients in dry powder form:

| | 51 % | 52 % | 53 % |
|---|---|---|---|
| Adrenaline | 0.1 | 0.1 | 0.1 |
| NaCl | 31.33 | 14.8 | 15 |
| Glucose | 50.4 | 66.77 | 60.9 |
| Glycine | 10.11 | 9.39 | 12 |
| Citric acid | 3.00 | 1.33 | 3 |
| $K_3$ citrate | 5.06 | 1.23 | 3 |
| $KH_2PO_4$ | -- | 6.38 | 6 |

Biological Evaluation

The following tests were carried out:

1. Mice

7-9 day old infant mice are separated from their mothers shortly before use and are administered the compound 45 mins prior to oral challenge with 0.05-0.10 ml of culture filtrate prepared from an enteropathogenic strain of *E. coli*. Control animals receive drug vehicle 45 mins prior to challenge with a similar amount of culture filtrate. The compounds are administered orally. Animals are killed two hours later and the entire intestine removed. The ratio of gut weight to remaining bodyweight (GW/BW) is determined from each animal and the increase in this ratio is determined by subtracting 0.06 (GW/BW for untreated mice) from the GW/BW of the animal. Drug treated animals are compared with untreated controls. If the compound has had an effect in inhibiting the fluid secretion caused by the enterotoxin(s) present in the culture filtrate then the gut weight/bodyweight ratio should be reduced in the treated animals. The percentage fluid inhibition is determined from the formula:

$$100 - \left[\frac{\text{Mean increase in GW/BW ratio in treated animals}}{\text{Mean increase in GW/BW ratio in control animals}} \times 100\right]$$

Results are given in the table below.

Results

| Compound | dose mg/kg p.o. | % Inhibition | Significance (Students t-test) |
|---|---|---|---|
| Adrenaline | 100 | 101 | $p<0.001$ |
| | 20 | 69 | $p<0.001$ |
| | 10 | 54 | $p<0.001$ |
| | 5 | 54 | $p<0.05$ |
| | 1 | 18 | |
| | 0.5 | 8 | |
| Adrenaline Bitartrate | 100 | 96 | $p<0.001$ |
| | 20 | 70 | $p<0.001$ |
| | 10 | 58 | $p<0.001$ |
| | 5 | 36 | $p<0.01$ |
| | 1 | 37 | $p<0.01$ |
| Noradrenaline (in 6mM ascorbate) | 10 | 40 | $p<0.05$ |
| Metoraminol | 200 | 45 | $p<0.05$ |

2. Calf Thiry-Vella Intestinal Loop Model

*In vivo* tests were conducted using male castrate calves, each with two surgically prepared Thiry-Vella intestinal loops prepared as described by R.J. Bywater, J. Comp. Path., 80, 565, (1970).

The loops are washed with saline and then a saline bolus is left in the loops for 30 minutes to establish a basal absorptive rate. After 30 minutes the fluid in the loops is removed and measured. Heat stable *E. coli* enterotoxin from *E. coli* strain p16 is added to the loop infusate which is then returned to the loops.

After a further 30 minutes the content of the loops is measured once more and at this time drug is added to the test loop perfusate.

Measurements of absorpotion or secretion from the loops are made every 30 minutes over the subsequent 2½ hours.

The results are expressed in terms of fluid (ml) secreted into or absorbed from the loops after each 30 minute period. A minimum of six observations are undertaken with each experimental compound. Perfusion periods in which drugs are present in a loop are alternated with perfusion periods in which drug is absent from the same loop.

<u>Calf-Thiry Vella Intestinal Loop Model</u>

A1 Adrenaline, 100 µg

| Time (min) (after toxin administration) (a) | Secretion/(ml/30 min) (mean ± SEM [n=10) | |
|---|---|---|
| | Control | 100 µg/kg Adrenaline |
| -30-0 | 3 ± 1 | 3 ± 1 |
| 0-30 | 27 ± 2 | 31 ± 3 |
| 30-60 | 18 ± 2 | 10 ± 1 * |
| 60-90 | 15 ± 2 | 8 ± 2 * |
| 90-120 | 10 ± 2 | 6 ± 2 |
| 120-150 | 12 ± 2 | 8 ± 3 |
| 150-180 | 9 ± 1 | 7 ± 2 |

* Denotes significantly different fluid secretion from control ($P<0.05$ students t test)

(a) Drug administered 30 min. after toxin

A2 Adrenaline bitartrate 30 µg/kg

| Time (min) after toxin administration (a) | Secretion (ml/30 min) mean ± SEM | |
|---|---|---|
| | Control | 30 µg/kg Adrenaline bitartrate |
| -30-0 | 4 ± 1 | 7 ± 2 |
| 0-30 | 17 ± 2 | 19 ± 3 |
| 30-60 | 12 ± 2 | 7 ± 3 |
| 60-90 | 10 ± 2 | 6 ± 1 |
| 90-120 | 8 ± 2 | 8 ± 2 |
| 120-150 | 8 ± 2 | 9 ± 3 |
| 150-180 | 8 ± 3 | 7 ± 3 |

B Novadrenaline 1 µg/kg

| Time (min) after toxin administration (a) | Secretion (ml/30 min) mean ± SEM | |
|---|---|---|
| | Control | 1 µg/kg Novadrenaline |
| -30-0 | 3 ± 2 | 2 ± 2 |
| 0-30 | 29 ± 3 | 30 ± 4 |
| 30-60 | 19 ± 2 | 16 ± 2 |
| 60-90 | 16 ± 3 | 15 ± 2 |
| 90-120 | 15 ± 1 | 5 ± 4 |
| 120-150 | 8 ± 1 | 5 ± 3 |
| 150-180 | 8 ± 2 | 11 ± 3 |

(a) Drug administered 30 minutes after toxin

3. Rabbits

Infant rabbits 7 - 10 days old are dosed with the compound under investigation orally 45 min prior to oral challenge with 50 ml/kg bodyweight of material prepared by cell lysis of an enteropathogenic strain of *E. coli*. Control animals receive drug vehicle 45 mins prior to challenge with a similar volume of material. 5 - 7 hours after oral administration of the challenge the animals are killed and gut weight/remaining bodyweight ratios calculated and the percentage fluid inhibition determined as above. Results are given in Table 3.

4. Piglets

2 - 4 Day old piglets are dosed with the compound orally 45 min prior to oral challenge with 25 ml of culture filtrate prepared from an enteropathogenic strain of *E. coli*. Control animals receive drug vehicle 45 min prior to challenge with a similar volume of material. Animals are observed for diarrhoea over a 7 hour period and the severity of scour scored on a 0 - 3 basis for each animal at hourly intervals. The percentage inhibition in treated animals is determined as:

$$100 - \left[\frac{\text{Mean score of scour in treated animals}}{\text{Mean score of scour in control animals}} \times 100\right]$$

Results obtained are given in Table 4.

Table 3 Activity of Adrenaline, in Rabbits, against heat labile toxin

| Drug | Dose mg/kg | % Inhibition of fluid secretion | Significance (P) |
|---|---|---|---|
| Adrenaline bitartrate | 20 | 43 | $< 0.001$ |
| | 10 | 23 | $< 0.05$ |
| | 5 | -3 | - |

Table 4: Effect of Adrenaline Bitartrate on Piglet Diarrhoea Induced by Enterotoxin Administration (Groups of 3 Piglets)

| Dose po. (mg/kg) | Duration of test (h.) | Mean Scour Score $\pm$ SEM | | % Inhibition of Scour | Significance (P) |
|---|---|---|---|---|---|
| | | Placebo treated | drug treated | | |
| 10 | 12 | 2.1 $\pm$ 0.2 | 0 $\pm$ 0 | 98 | $<0.01$ |
| 2 x 5 | 12 | 2.1 $\pm$ 0.2 | 0.1 $\pm$ 0.1 | 96 | $<0.01$ |
| 1 | 6 | 2.3 $\pm$ 0.2 | 2.0 $\pm$ 0.2 | 11 | |
| 1 | 12 | 2.1 $\pm$ 0.0 | 1.8 $\pm$ 0.1 | 16 | |

Toxicity

In mice the drugs have been found to have a satisfactory therapeutic ratio; no untoward effects being observed when administered at 100 mg/kg per os. 1 mg/kg b.i.d. for 7 days is well tolerated by neonatal piglets and 2 mg/kg b.i.d. for 4 days in calves under 50 kg.

Claims

1. An oral rehydration formulation comprising a pharmacologically acceptable aqueous solution containing at least 0.5% w/v of an actively absorbed monosaccharide, at least 25 mM sodium ions and having an osmolarity less than 500 mOsmolar characterised in that the formulation also comprises a sympathomimetic phenylethylamine derivative having $\alpha_2$-adrenoceptor agonist activity.

2. A formulation as claimed in claim 1 wherein the phenylethylamine derivative is a compound of formula (I)

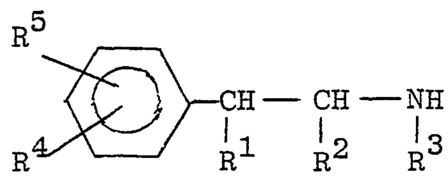

(I)

wherein $R^1$ is hydrogen or hydroxy
$R^2$ is hydrogen or methyl
$R^3$ is hydrogen or methyl
$R^4$ is hydrogen, meta-hydroxy or ortho-methoxy
and $R^5$ is hydrogen, para-hydroxy or meta-methoxy
or a salt thereof.
provided that the compound has some $\alpha_2$-adrenergic agonist activity.

3. A formulation as claimed in claim 2 wherein the compound of formula (I) is selected from

   epinephrine
   norepinephrine
   nordefrin
   metaraminol
   phenylephrine
   hydroxyamphetamine
   methoxamine
   methamphetamine
   ephidrine
   phenylpropanolamine
   and mephentermine and salts thereof.

4. A formulation as claimed in claim 2 wherein the compound of formula (I) is epinephrine, norepinephrine or metaraminol or a salt thereof.

5. A formulation as claimed in any one of claims 1 to 4 wherein the phenylethylamine derivative is present in a concentration of from 0.01 g/litre to 0.2 g/litre.

6. A formulation as claimed in any one of claims 1 to 5 which further comprises an actively absorbed amino acid and an electrolyte.

7. A formulation as claimed in claim 6 which comprises an actively absorbed monosaccharide selected from glucose, galactose, α-methyl glucoside, 3-O-methyl glucose and 6-deoxygalactose,

   an actively absorbed amino acid selected from glycine, alanine and arginine,

an electrolyte, selected from potassium dihydrogen phosphate, dipotassium hydrogen phosphate, tripotassium phosphate, sodium, potassium, calcium or magnesium chloride, sodium, potassium, calcium or magnesium gluconate, sodium, potassium, calcium or magnesium phosphate, sodium, potassium, calcium or magnesium sulphate, sodium, potassium, calcium or magnesium bicarbonate, and sodium, potassium, calcium or magnesium carbonate.

8. A dry powder for dissolution in water, which powder comprises an actively absorbed monosaccharide, sodium ions and a sympathomimetic phenylethylamine derivative having $\alpha_2$-adrenoceptor agonist activity in amounts suitable to provide a formulation as claimed in claim 1 on dissolution in water.

9. A process for producing a formulation as claimed in claim 1 comprising bringing into association the actively absorbed monosaccharide, sodium ions and phenylethylamine derivative.

European Patent Office

# EUROPEAN SEARCH REPORT

0059057

Application number

EP 82 30 0758

| Category | Citation of document with indication, where appropriate, of relevant passages | Relevant to claim | CLASSIFICATION OF THE APPLICATION (Int. Cl. 3) |
|---|---|---|---|
| D,A | GB-A-2 012 163 (BEECHAM GROUP LTD.)<br>--- | 1-9 | A 61 K 31/70<br>A 61 K 33/00 //<br>(A 61 K 33/00<br>A 61 K 33/70<br>A 61 K 31/195<br>A 61 K 31/135) |
| D,A | GB-A-1 581 826 (BEECHAM GROUP LIMITED)<br>--- | 1-9 | |
| D,A | US-A-3 898 328 (MYRON A.BEIGLER)<br>--- | 1-9 | |
| A | MARTIN NEGWER: "Organisch-Chemische Arzneimittel und ihre Synonyma", vol. I, 1978, page 199,AKADEMIEVERLAG, Berlin, DE. *Nr. 1083 ADRENALIN*<br>----- | 1-9 | |

DOCUMENTS CONSIDERED TO BE RELEVANT

TECHNICAL FIELDS SEARCHED (Int. Cl. 3)

A 61 K

The present search report has been drawn up for all claims

| Place of search | Date of completion of the search | Examiner |
|---|---|---|
| THE HAGUE | 16-04-1982 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document